# FASCICULE DE BREVET EUROPEEN

(11) **EP 2 119 352 B1**
(45) Date de publication et mention de la délivrance du brevet: **12.04.2017**
(21) Numéro de dépôt: 09290344.2
(22) Date de dépôt: 11.05.2009
(51) Int. Cl.: A01N 1/02, C12M 1/00

(54) **Procédé de conservation de cellules sécrétrices d'insuline destinées à être transplantées**
Verfahren zur Konservierung von insulinsekretierenden Zellen, die für die Transplantation bestimmt sind
Method for conserving insulin-secreting cells intended for transplant

(30) Priorité: 16.05.2008 FR 0802699
(43) Date de publication de la demande: 18.11.2009
(73) Titulaire: MACO PHARMA, 59420 Mouvaux (FR); Centre Hospitalier Regional Universitaire de Lille, 59037 Lille Cédex (FR); UNIVERSITE DE DROIT ET DE LA SANTE DE LILLE 2, 59800 Lille (FR)
(72) Inventeur: Pattou, François, 59000 Lille (FR); Kerr-Conte, Julie, 59000 Lille (FR); Lukowiak, Bruno, 62690 Aubigny en Artois (FR); Coissac-Blondel, Cécile, 59000 Lille (FR); Vandewalle, Brigitte, 59000 Lille (FR); Heron, Antoine, 59250 Halluin (FR)
(74) Mandataire: Sayettat, Julien Christian

(56) Documents cités:
- EP-A- 1 875 802
- EP-A- 1 903 101
- WO-A-2005/120576
- US-A1- 2006 246 582

## Description

L'invention concerne un procédé de conservation d'îlots pancréatiques destinés à être transplantés à un patient.

L'invention s'applique au domaine de la thérapie cellulaire du diabète qui vise à préparer des îlots pancréatiques ou îlots de Langerhans à partir de pancréas prélevés sur un donneur en état de mort encéphalique. Ces îlots qui comprennent des cellules bêta sécrétrices d'insuline sont ensuite réinjectés dans la veine porte d'un malade receveur dans le but d'obtenir de nouveau chez ce malade, une régulation de sa glycémie sans recours aux injections quotidiennes d'insuline recombinante.

Cette thérapie cellulaire représente donc une alternative intéressante à la greffe de pancréas, avec une chirurgie moins lourde et moins de complications.

Pratiquement, les îlots pancréatiques sont isolés par digestion enzymatique et mécanique à partir d'un pancréas d'un donneur, puis purifiés par gradient de densité. Les îlots sont ensuite perfusés directement au patient ou bien cultivés pendant 1 à 3 jours avant leur transplantation.

Cette étape de culture ou conservation pré-transplantation s'est avérée bénéfique sur le plan métabolique et immunologique. En effet, le temps passé à cultiver les îlots peut permettre de préparer le receveur à la greffe. En outre, cette étape de culture permet également de réaliser les contrôles qualité sur les îlots nécessaires avant leur perfusion.

Cependant, avec les méthodes actuelles de conservation statique, entre 40 et 60% des îlots sont perdus pendant la première période de 24 heures de culture après isolement.

De plus, la culture des îlots s'effectue en milieu ouvert dans des récipients standards (flasques ou boîtes de pétri) au prix de nombreuses manipulations, qui entraînent un risque de contamination virale ou bactérienne des îlots.

De nombreuses recherches sont menées sur la composition du milieu de culture dans le but d'améliorer la conservation des îlots en culture.

Par exemple, le document WO2005/120576 propose d'ajouter 50 M d'alpha-tocophérol dans un milieu de culture pour réduire les dommages causés aux îlots pancréatiques par anoxie. Le document US2007/0196810 propose d'ajouter une hémoglobine polymérisée au milieu de culture utilisé pour isoler les îlots du pancréas. Et, dans le document US2006/0246582, un peptide analogue à la chaîne de la laminine A est ajouté au milieu de culture des îlots pour permettre d'augmenter la densité de culture des îlots jusqu'à 300 îlots/mL.

Ces milieux restent cependant insuffisants pour obtenir un taux de survie acceptable des îlots en culture.

Quant au conditionnement des îlots, il a été envisagé dans les documents EP-A1-1875802 et US-2005/0032205 d'utiliser une poche souple perméable à l'oxygène pour cultiver les îlots. Le taux de survie des îlots dans ces poches souples reste également insuffisant.

Le document EP-1 903 101 concerne un procédé de renouvellement d'un milieu biologique dans lequel des cellules sont en suspension, ledit procédé prévoyant de :
- mettre en communication la suspension avec une source de milieu biologique de renouvellement par l'intermédiaire d'une voie de communication, ladite voie comprenant un moyen de rétention des cellules et, entre le moyen de rétention et la source, un moyen d'évacuation du milieu biologique ;
- évacuer dans le moyen d'évacuation le milieu biologique de la suspension par passage au travers du moyen de rétention dans un premier sens ; et
- transférer le milieu de renouvellement au travers du moyen de rétention dans un deuxième sens, pour mettre en suspension les cellules avec le milieu de renouvellement, ledit deuxième sens étant prévu pour renvoyer dans la suspension les éventuelles cellules retenues dans le dispositif de rétention.

Par ailleurs, le document EP-1 875 802 concerne un procédé de conservation dans un réservoir d'un liquide contenant des îlots pancréatiques, dans lequel au moins une paroi du réservoir consiste en un film avec un coefficient de perméabilité à l'oxygène d'au moins 2500 cm³/m².jour.atm et/ou avec un coefficient de perméabilité au dioxyde de carbone compris entre 1000 et 20000 cm³/m².jour.atm.

L'invention propose donc un procédé pour conserver les îlots pancréatiques, permettant d'atteindre un taux de survie supérieur à 60% jusqu'à 72 heures et de maintenir la fonctionnalité des îlots, c'est-à-dire leur capacité à produire de l'insuline une fois réimplantés chez le receveur. De plus, la méthode est réalisée dans des conditions aseptiques réduisant le risque de contamination bactérienne et virale.

A cet effet, l'invention propose un procédé de conservation d'îlots pancréatiques destinés à être transplantés, comprenant les étapes suivantes :
- l'introduction d'un volume initial de milieu de culture et d'îlots pancréatiques dans un récipient de culture,
- la provision d'une source de milieu de culture,
- le remplacement à des intervalles de temps inférieurs à 8 heures de milieu de culture dudit récipient de culture par du milieu de culture provenant de ladite source de sorte à renouveler le milieu de culture contenu dans ledit récipient, ledit procédé permettant d'atteindre un taux de survie supérieur à 60% jusqu'à 72 heures.

D'autres objets et avantages apparaîtront au cours de la description qui suit.
La figure 1 représente de façon schématique un dispositif pour mettre en oeuvre un procédé de l'invention selon lequel un flux continu est établi dans le récipient de culture.
La figure 2 représente de façon schématique un dispositif pour mettre en oeuvre un procédé de l'invention selon lequel le milieu de culture qui a été préalablement introduit dans le récipient de culture est en partie recyclé.
La figure 3 représente de façon schématique un dispositif pour mettre en oeuvre un procédé de l'invention selon lequel le milieu de culture est oxygéné préalablement à son introduction dans le récipient de culture.
Les figures 4 à 11 représentent l'évaluation d'îlots pancréatiques conservés selon un procédé de l'invention dans le milieu de culture M1 ou le milieu de culture M2.

Notamment, les îlots pancréatiques sont issus d'un pancréas d'un donneur en état de mort encéphalique. Ils sont isolés par digestion enzymatique et mécanique du pancréas puis purifiés par gradient de densité. Ils sont ensuite conservés avant d'être transplantés.

Le procédé de l'invention s'applique aux îlots ainsi isolés contenus dans des préparations de haute pureté, notamment de l'ordre de 90%, mais aussi à des préparations de pureté moindre telle qu'inférieure à 50%. Même dans le cas où la pureté des préparations d'îlots n'est pas satisfaisante, le procédé de l'invention permet d'obtenir un taux de survie des îlots supérieur à 80%, jusqu'à 72 heures.

Selon l'invention, le procédé de conservation d'îlots pancréatiques comprend les étapes suivantes :
- l'introduction d'un volume initial de milieu de culture et d'îlots pancréatiques dans un récipient de culture,
- la provision d'une source de milieu de culture,
- le remplacement à des intervalles de temps inférieurs à 8 heures de milieu de culture dudit récipient de culture par du milieu de culture provenant de ladite source de sorte à renouveler le milieu de culture contenu dans ledit récipient, ledit procédé permettant d'atteindre un taux de survie supérieur à 60% jusqu'à 72 heures.

Selon un premier aspect, le renouvellement de milieu est périodique, c'est-à-dire le remplacement de milieu de culture contenu dans le récipient est réalisé à des intervalles de temps déterminés, généralement réguliers.

Par exemple, du milieu de culture contenu dans le récipient est remplacé toutes les 2 heures. En particulier, du milieu de culture est d'abord retiré du récipient de culture, puis du milieu de culture provenant de la source est introduit dans le récipient. Lorsque le récipient est muni d'un orifice d'entrée et d'un orifice de sortie distincts, ces étapes de retrait et d'introduction peuvent être simultanées.

Selon un deuxième aspect, le renouvellement de milieu est continu : du milieu de culture contenu dans le récipient est remplacé de façon ininterrompue par du milieu de culture provenant de la source de milieu. L'intervalle de temps est dans ce cas nul.

Ce renouvellement, périodique ou continu, est notamment précoce, c'est-à-dire qu'il est réalisé dès la mise en culture des îlots après leur isolement. En renouvelant le milieu toutes les deux heures, les enzymes libérées par le tissu exocrine sont éliminées du récipient de culture, procurant aux îlots des conditions de conservation plus favorable. En outre, le renouvellement régulier du milieu de culture permet un apport régulier en oxygène, ce qui favorise également la conservation des îlots.

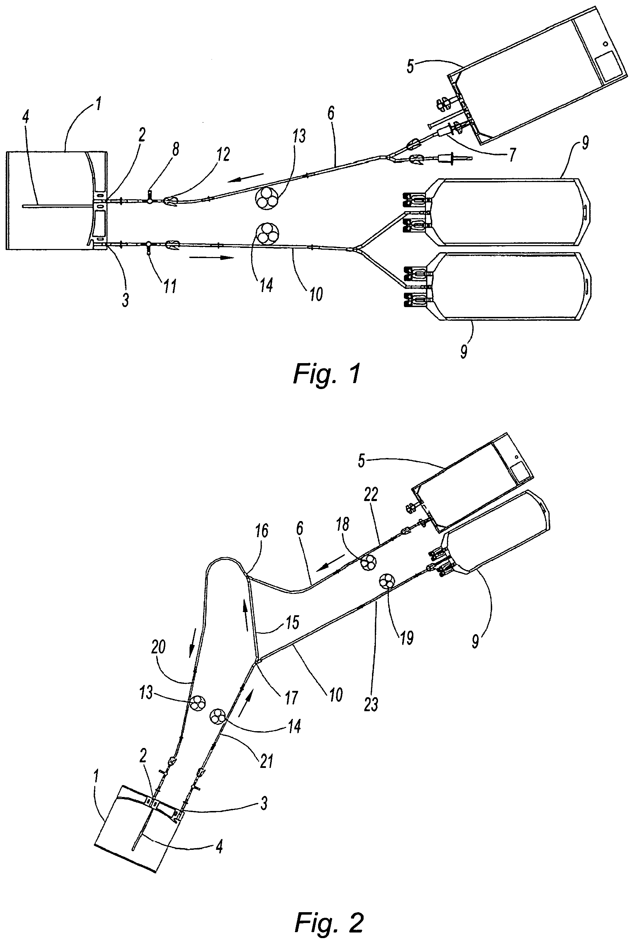

Selon la figure 1 et lorsque le renouvellement du milieu est réalisé en continu, le récipient de culture 1 comprend au moins un orifice d'entrée 2 et au moins un orifice de sortie 3 et le remplacement du milieu de culture est réalisé par établissement d'un flux continu de milieu de culture provenant de la source 5 de milieu, ledit flux alimentant le récipient de culture 1 par l'intermédiaire de l'orifice d'entrée 2 et étant retiré dudit récipient de culture par l'intermédiaire de l'orifice de sortie 3, de sorte à renouveler en continu le milieu de culture contenu dans ledit récipient.

Ainsi, lorsque le flux continu est établi, il se crée une circulation de milieu dans le récipient de culture, de sorte que lorsque du milieu est introduit dans le récipient de culture, du milieu de culture est simultanément retiré du récipient de culture.

Le renouvellement en continu du milieu entraîne une dilution des enzymes libérées par le tissu exocrine et réduit ainsi les concentrations enzymatiques délétères pour les îlots.

La concentration des îlots pancréatiques dans le milieu de culture est comprise entre 400 et 40 000 îlots équivalents/ mL. Avantageusement, la concentration des îlots est de l'ordre de 500-3 000 îlots équivalents /mL.

Du fait que les îlots peuvent avoir des tailles très différentes et qu'ils ont tendance à s'agglomérer entre eux, il est difficile de définir une concentration d'îlots. On préfère ainsi parler d'îlots équivalents (IE), un îlot équivalent correspondant à un îlot théorique de 150 µm de diamètre.

Cette concentration d'îlots relativement élevée par rapport aux méthodes actuelles de conservation permet d'utiliser pour la conservation des récipients de culture de taille acceptable pour une plus grande maniabilité et un gain d'espace.

Le milieu de culture des îlots pancréatiques utilisé dans le procédé de l'invention comprend notamment un milieu de base supplémenté en albumine. Par exemple, le milieu de base est le milieu CMRL-1066. De façon avantageuse, l'albumine est de l'albumine humaine naturelle ou recombinante.

La durée d'établissement du flux de milieu est supérieure à 24 heures, notamment comprise entre 24 heures et 72 heures. Cette durée est suffisante pour réaliser en parallèle des contrôles qualité sur un échantillon de cellules sécrétrices d'insuline et pour préparer le receveur à la transplantation.

En outre, il est possible que cette étape de conservation sous condition de flux possède un effet positif sur la qualité des îlots.

Plus précisément, le débit du flux alimentant l'orifice d'entrée du récipient de culture est compris entre 0,2 et 5 mL/minute, notamment 1,5 mL/minute. Ce débit est avantageusement uniforme pendant la durée d'établissement du flux continu.

Comme les îlots ne doivent pas adhérer à la surface du récipient de culture pour ne pas perdre de leur fonctionnalité, le débit doit être choisi suffisamment faible pour ne pas entraîner les îlots dans le flux de milieu en dehors du récipient, mais suffisamment élevé pour permettre un renouvellement suffisant du milieu de culture.

En effet, une préparation d'îlots n'est pas pure à 100%. Elle comporte une fraction importante de tissu exocrine, capable de relarguer un grand nombre de molécules dont des protéases. Il est possible que ce tissu exocrine soit délétère pour les îlots et contribue majoritairement à leur dégradation. Ainsi, un renouvellement du milieu limite la présence de protéases et prévient la mortalité cellulaire.

En outre, il est reconnu, par exemple du document EP-A1-1875802 que l'oxygène est bénéfique à la survie et la fonctionnalité des îlots. Le renouvellement continu du milieu de culture contribue également à apporter de l'oxygène aux îlots et favorise leur survie.

En particulier, le récipient de culture contenant les îlots est placé à une température comprise entre 20°C et 40°C, notamment 37°C. Pour ce faire, le récipient, et éventuellement les autres récipients et tubulures associés, sont placés dans un incubateur.

En relation avec la figure 1 et selon un aspect particulier de l'invention, le récipient de culture 1 comprend une poche souple biocompatible réalisée en matière thermoplastique. Notamment, le matériau de la poche en contact avec la suspension d'îlots pancréatiques empêche l'adhésion des îlots. Par exemple, la poche souple est réalisée en éthylène - acétate de vinyle.

La concentration et le nombre d'îlots à conserver déterminent la taille du récipient de culture. Des poches souples ayant une surface comprise entre 250 et 800 cm², notamment 400 cm² sont utilisées. Par exemple, les poches possèdent une taille de 19 cm x 20,5 cm pour obtenir une surface de 389 cm². Selon un autre exemple, on utilise des poches de 18,5 cm x 37 cm pour obtenir une surface de 685 cm².

Le récipient de culture 1 comprend au moins un orifice d'entrée 2 pour l'alimentation en milieu de culture du récipient et un orifice de sortie 3 pour le retrait du milieu de culture du récipient.

Par exemple, les orifices d'entrée et sortie 2,3 de la poche sont formés par des portions de tubulures disposées à la périphérie de la poche, entre les deux faces de la poche.

Selon la figure 1, les orifices d'entrée et sortie 2,3 sont disposés sur un même côté de la poche souple.

Dans une réalisation particulière, pour faciliter la circulation du milieu de culture dans le récipient celui-ci comprend au moins un déflecteur 4 disposé sur le chemin d'écoulement direct entre l'orifice d'entrée 2 et l'orifice de sortie 3 du récipient de culture 1.

Lorsque le récipient 1 est sous la forme d'une poche en un matériau souple, le déflecteur 4 est notamment sous forme d'une soudure s'étendant perpendiculairement depuis le côté sur lequel sont disposés les orifices d'entrée et sortie 2,3, ladite soudure étant formée entre lesdits orifices.

Ainsi, le flux est forcé à pénétrer jusque au voisinage du fond de la poche de sorte que la totalité du milieu de culture puisse circuler dans la poche et que les îlots soient alimentés régulièrement en milieu.

Lors de la mise en oeuvre du procédé, l'orifice d'entrée 2 de la poche est en communication fluidique avec un récipient 5 formant une source de milieu de culture, par l'intermédiaire d'une première tubulure 6.

Par exemple, le récipient formant une source de milieu de culture, dit récipient source, comprend une poche souple, un flacon ou une bouteille contenant un milieu de culture. En variante, la source de milieu de culture comprend une pluralité de récipients de culture 5 en communication fluidique avec le récipient de culture 1.

Pour permettre la connexion avec le récipient 5 formant une source de milieu de culture, l'extrémité de la première tubulure 6 comprend un connecteur 7 tel qu'un perforateur.

Selon une réalisation, la première tubulure 6 est munie d'un dispositif d'introduction des îlots 8. Par exemple, le dispositif d'introduction est sous la forme d'un connecteur trois voies, notamment un robinet à trois voies. Avantageusement, le dispositif d'introduction 8 est placé le plus proche possible du récipient de culture 1 pour éviter une perte d'îlots dans la tubulure. Encore plus avantageusement, le dispositif d'introduction 8 des îlots est sous forme d'un deuxième orifice d'entrée du récipient de culture 1.

L'orifice de sortie 3 du récipient de culture 1 est en communication fluidique avec un récipient 9 destiné à contenir le milieu de culture retiré par l'orifice de sortie 3, par l'intermédiaire d'une deuxième tubulure 10.

Ce récipient 9 destiné à contenir le milieu de culture retiré par l'orifice, dit récipient poubelle, est sous forme d'une poche souple, d'un flacon ou d'une bouteille.

En variante, plusieurs récipients poubelle 9 sont en communication fluidique avec le récipient de culture 1.

Selon une réalisation, la deuxième tubulure 10 est munie d'un dispositif de retrait 11 des îlots. Ce dispositif de retrait est identique ou différent du dispositif d'introduction des îlots et se présente sous la forme d'un connecteur trois voies, notamment un robinet trois voies. Comme le dispositif d'introduction, le dispositif de retrait est placé le plus proche possible du récipient de culture pour éviter de laisser des îlots dans la tubulure lors du retrait.

Notamment, les îlots pancréatiques sont retirés du récipient de culture 1 par aspiration dans une seringue. Dans une variante, ils s'écoulent par gravité dans un récipient de récolte.

En variante non représentée, le dispositif de retrait 11 est agencé sur le récipient de culture et forme un deuxième orifice de sortie.

Selon une réalisation particulière, les première et deuxième tubulures 6,10 sont pourvues d'au moins un dispositif de régulation 12 du flux tel qu'un clamp. Les tubulures sont réalisées en un matériau thermoplastique soudable et stérilisable tel que le polychlorure de vinyle.

Pour établir un flux continu de milieu de culture dans ledit récipient de culture 1, une première tête de pompe péristaltique 13 est associée à la première tubulure 6 de sorte à alimenter l'orifice d'entrée 2 du récipient de culture 1 avec le flux de milieu provenant du récipient source 5.

Avantageusement, une deuxième tête de pompe péristaltique 14 est en plus associée à la deuxième tubulure 10 de sorte à retirer du récipient 1 le flux de milieu de culture par l'orifice de sortie 3.

Selon une réalisation particulière non représentée, une pompe péristaltique à deux têtes de pompe est utilisée.

Lorsque le flux dans le récipient de culture 1 est établi à l'aide de cette première et deuxième tête de pompe péristaltique 13,14 ayant une vitesse de rotation identique, le volume de milieu de culture dans le récipient est constant.

Par contre, lorsque la vitesse de rotation de la première tête de pompe péristaltique 13 est supérieure à la vitesse rotation de la deuxième tête de pompe péristaltique 14, le volume de milieu de culture dans le récipient augmente.

Selon un aspect particulier du procédé, le procédé de conservation des îlots comprend, après l'introduction des îlots dans le récipient de culture 1, une phase stationnaire dans laquelle le flux est agencé pour maintenir constant le volume de milieu de culture contenu dans le récipient 1.

Préalablement à la phase stationnaire, et après l'introduction des îlots dans le récipient 1, le flux est agencé pour augmenter le volume initial du milieu de culture dans le récipient de culture 1.

Par exemple, 30 mL de milieu de culture et 45000 îlots équivalents sont introduits dans une poche souple ayant une surface de 389 cm². La première tête de pompe 13 est mise en rotation à une vitesse de 1 mL/min tandis que la deuxième tête de pompe 14 est arrêtée. Lorsque le volume dans la poche est d'environ 90 mL, la deuxième tête de pompe 14 est mise en rotation à une vitesse identique à la première tête de pompe pour maintenir le volume de milieu dans la poche constant.

La vitesse de rotation des deux têtes de pompes est notamment choisie de sorte que la quantité de milieu de culture provenant continûment de la source de milieu pendant 2 heures équivaut à ladite quantité utilisée lors d'un renouvellement périodique du milieu toutes les 2 heures. Par exemple, si 15 mL de milieu est remplacé périodiquement toute les 2 heures, la vitesse de rotation est choisie pour que 15 mL de milieu provenant de la source soit introduit dans le récipient au bout de 2 heures.

Selon une variante de l'invention représentée sur la figure 2, une partie au moins du flux de milieu de culture est recyclée, c'est-à-dire qu'au moins une partie du flux de milieu de culture alimentant l'orifice d'entrée 2 provient du flux de milieu de culture qui a été préalablement retiré par l'orifice de sortie 3.

Cette variante permet d'économiser le milieu de culture tout en continuant à renouveler l'oxygène du récipient de culture.

Le procédé permet également de réduire la concentration en dioxyde de carbone qui est délétère pour les îlots et de diminuer la concentration des enzymes libérées par le tissu exocrine.

Dans cette variante et selon la figure 2, une troisième tubulure 15 est branchée entre les première et deuxième tubulures 6,10. Notamment, le branchement est réalisé à l'aide d'un premier et deuxième dispositif de connexion 16,17 pourvu respectivement sur la première et la deuxième tubulure 6,10. Notamment, le dispositif de connexion 16,17 est de type connecteur trois voies, tel que connecteur en T ou Y.

Selon une réalisation particulière, la troisième tubulure 15 est branchée sur la première tubulure 6 en amont de la première tête de pompe 13 et sur la deuxième tubulure 10 en aval de la deuxième tête de pompe 14. En outre, une troisième tête de pompe péristaltique 18 est associée sur la première tubulure, près du récipient source 5, en amont du branchement 16 avec la troisième tubulure 15 et une quatrième tête de pompe péristaltique 19 est associée, près du récipient poubelle, à la deuxième tubulure 10 en aval du branchement 17 avec la troisième tubulure 15.

Les termes "amont" et "aval" sont ici définis en relation avec le sens de circulation du milieu dans les tubulures et les récipients de culture.

Notamment, une deuxième pompe péristaltique comprend la troisième et quatrième tête de pompe 18,19.

Avantageusement, les première, deuxième, troisième et quatrième têtes de pompe 13,14,18,19 appartiennent à une même pompe péristaltique pouvant contrôler la vitesse de rotation de chacune des têtes de pompe.

La portion de tubulure 20,21,22,23 associée à une tête de pompe 13,14,18,19, est notamment réalisée en matière siliconée plus souple que le PVC.

Selon la figure 2 et pour assurer le recyclage du milieu de culture, la troisième et quatrième tête de pompe 18,19 ont une vitesse de rotation identique, et inférieure à la vitesse de rotation de la première et deuxième tête de pompe.

Par exemple, la vitesse de rotation de la première et deuxième tête de pompe 13,14 est de 1,5 mL/minute tandis que la vitesse de rotation de la troisième et quatrième tête de pompe 18,19 a une vitesse de rotation de 0,3 mL/min.

En particulier, le flux de milieu de culture est oxygéné préalablement à son alimentation dans l'orifice d'entrée du milieu de culture.

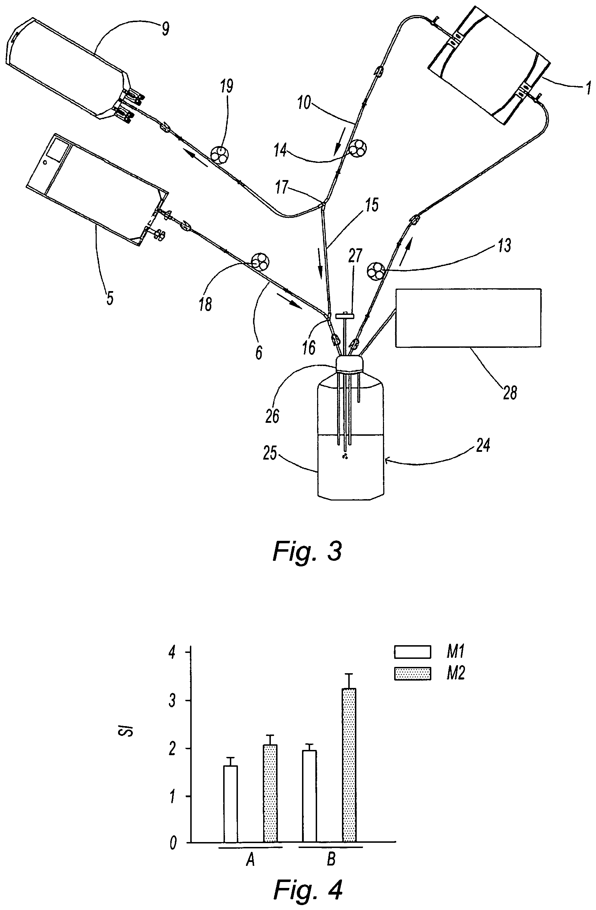

Pour ce faire et comme représenté sur la figure 3, un système d'aération 24 du flux de milieu de culture est placé sur le chemin d'écoulement de la première tubulure 6.

Selon la variante représentée sur la figure 3, le système d'aération est situé entre le premier dispositif de connexion et la première tête de pompe péristaltique 13.

Le système d'aération comprend un récipient d'aération 25 telle qu'une bouteille, un dispositif d'introduction d'air 27 tel qu'une portion de tubulure ouverte sur l'air extérieur et munie d'un filtre de porosité moyenne de 0,22 µm, et d'une pompe à vide 28.

La bouteille est notamment munie d'un bouchon 26 pourvu de deux connecteurs pour son branchement sur la première tubulure 6, d'un connecteur pour le dispositif d'introduction de l'air 27 et d'un connecteur pour la pompe à vide 28.

Selon une variante non représentée, le système d'aération comprend un oxygénateur plongeant directement dans le récipient source 5.

Selon une réalisation particulière, le récipient de culture 1, les tubulures reliées 6,10 audit récipient et éventuellement le récipient poubelle 9 sont pré-connectés de fabrication et placés dans un emballage stérile. Au moment de l'utilisation, l'utilisateur n'a plus alors qu'à connecter le récipient source 5 et éventuellement le système d'aération 24 pour obtenir un système clos de culture répondant aux exigences en matière de réduction du risque de contamination.

Le dispositif ainsi obtenu permet une conservation en circuit clos et sécurisée des cellules sécrétrices d'insuline.

### Exemples

### Exemple 1

Des îlots pancréatiques ont été isolés et conservés par renouvellement régulier périodique du milieu de culture. Une circulation d'air associée à un renouvellement périodique du milieu (1/4 du volume par heure) a été envisagée. Les poches utilisées sont de l'ordre de 400 cm², soit proches de celles qui seront utilisées en usage clinique.

Les essais ont été réalisés sur deux préparations d'îlots différentes, la première (Poche 1) de pureté en îlots médiocre : 30 % et la seconde (Poche 2) de très bonne pureté : 90 %. Les pressions partielles en oxygène (PpO₂) sont supérieures dans les poches que dans les boîtes contrôles. Les résultats sont repris dans le tableau 1.

**Tableau 1**

| | Contrôle 1 | Poche 1 | Contrôle 2 | Poche 2 |
|---|---|---|---|---|
| Pureté | 30% | 30% | 90% | 90% |
| PpO₂ (mmHg) | 139 | 151 | 141 | 151 |

Le pourcentage de récupération des îlots a été amélioré dans les poches, il était de l'ordre de 20 % supérieur à celui obtenu dans les boîtes contrôles. Il était équivalent dans la préparation médiocre où la perte d'îlots est habituellement plus importante que dans les préparations plus pures.

### Exemple 2

Dans cet exemple, 15 000 îlots sont placés dans une boîte de culture avec 30 mL de milieu. Deux séries d'essais de conservation ont été effectuées, l'une contrôle sans renouvellement de milieu de culture (série A), l'autre avec renouvelant du milieu de culture (série B).

De plus, deux milieux de culture ont été testés, d'une part le milieu de culture M1 comprenant le milieu CMRL (In Vitrogen) supplémenté en albumine humaine, et d'autre part le milieu de culture M2 comprenant le milieu de base CMRL modifié (Cambrex) supplémenté en sérum AB et Stem ease® (Abcys). Le renouvellement a été réalisé en renouvelant 15mL de milieu toutes les 2 heures pendant 14 heures (à 2 heures, 4 heures, 6 heures, 8 heures, 10 heures et 14 heures après isolement). L'évaluation a été réalisée à 18h.

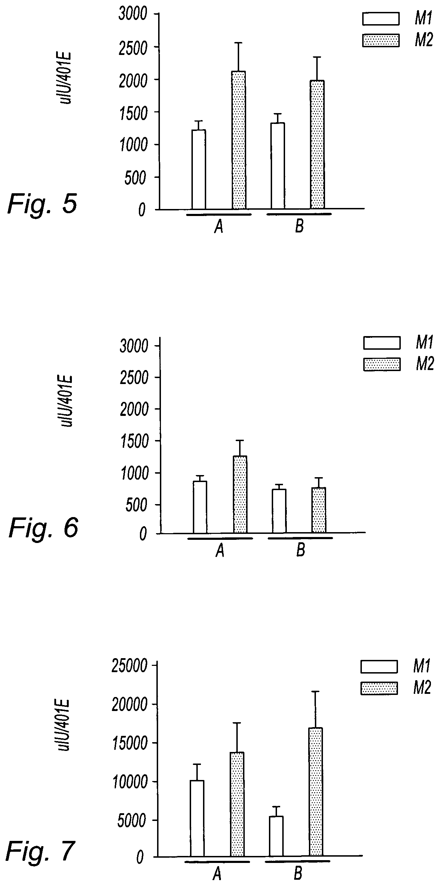

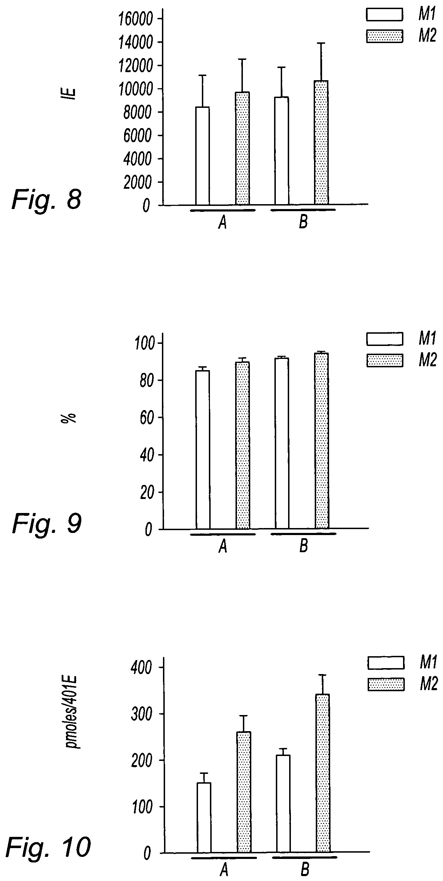

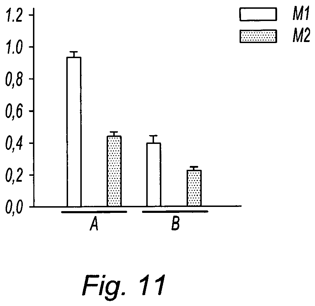

Les critères évalués ont été les suivants :
- le nombre d'îlots équivalents (IE) par comptage des îlots après coloration à la dithizone (figure 8),
- la physiologie des îlots par détermination de la quantité (µIU/40 IE) en insuline (figure 7), du taux de sécrétion d'insuline (µIU/40 IE) en réponse à 20 mM de glucose (figure 5) et à 2,8 mM de glucose (figure 6), et de l'index de stimulation (figure 4),
- la mort cellulaire par dosage (à 405 nm) de l'apoptose (figure 11),
- l'estimation de la viabilité des îlots en pourcentage (figure 9), et
- la détermination du contenu (pmoles/40IE) en adénosine triphosphate (ATP) (figure 10).

L'estimation microscopique classique de la viabilité, de la purification et du rendement cellulaires a été réalisée par coloration sélective des cellules bêta par la dithizone, marqueur spécifique de l'insuline intracellulaire, et par le bleu Trypan marqueur des cellules mortes. Les cellules bêta mortes apparaissent colorées en violet.

L'activité fonctionnelle des cellules bêta a été évaluée par les taux d'insuline intracellulaire et la sécrétion d'insuline en réponse à des tests de stimulation par le glucose.

Le stockage intracellulaire et la transmission de l'énergie sont essentiellement réalisés par la synthèse et la régénération de l'adénosine triphosphate (ATP). L'ATP contenu dans les îlots a été dosé sur 3 échantillons de 40 IE par pancréas, par la détermination en luminométrie de la réaction entre la luciférine et la luciférase à l'aide d'un kit commercial (Perkin Elmer).

L'apoptose est caractérisée par la fragmentation de l'ADN en mono et oligonucléosomes qui peuvent être détectés dans les lysats cellulaires. La quantification des nucléosomes a été réalisée à l'aide du kit « Cell Death Detection ELISA » de chez Roche Molecular Biochemicals. Le principe de détection est fondé sur un dosage immunoenzymatique sandwich associant des anticorps monoclonaux dirigés contre l'ADN et les histones. Le dosage a été réalisé sur 3 échantillons de 160 IE par pancréas.

Le contenu en insuline a été dosé par une technique radioimmunologique à l'aide du kit CT de CIS bio international sur les lysats de 3 échantillons de 40 IE par pancréas. Les sécrétions d'insuline ont été estimées pendant 2 périodes consécutives de une heure. La première en présence de 2,8 mM de glucose et la seconde à la concentration stimulante de 20 mM de glucose. L'index de stimulation est défini comme le rapport des quantités d'insuline sécrétées pendant ces deux périodes (forte sur faible concentration). Les sécrétions d'insuline ont été estimées sur 5 échantillons de 40 IE par pancréas.

Il résulte des essais (figures 4 à 11) que, lorsque le milieu est remplacé précocement et régulièrement :
- l'index de stimulation augmente de 20 à 30%,
- les îlots ne sont pas altérés et ne sécrètent pas excessivement d'insuline à 2,8 mM de glucose,
- la quantité d'ATP est plus importante,
- l'apoptose est diminuée.

## Revendications

1. Procédé de conservation d'îlots pancréatiques destinés à être transplantés, comprenant les étapes suivantes :
- l'introduction d'un volume initial de milieu de culture et d'îlots pancréatiques dans un récipient de culture (1),
- la provision d'une source (5) de milieu de culture,
- le remplacement à des intervalles de temps inférieurs à 8 heures de milieu de culture dudit récipient de culture par du milieu de culture provenant de ladite source de sorte à renouveler le milieu de culture contenu dans ledit récipient, ledit procédé permettant d'atteindre un taux de survie supérieur à 60% jusqu'à 72 heures.

2. Procédé selon la revendication 1, **caractérisé en ce que** la concentration des îlots pancréatiques dans le milieu de culture est comprise entre 400 et 40 000 îlots équivalents /mL.

3. Procédé selon l'une des revendications 1 ou 2, **caractérisé en ce que** le milieu de culture comprend un milieu de base supplémenté en albumine.

4. Procédé selon l'une quelconque des revendications 1 à 3, **caractérisé en ce que** du milieu de culture contenu dans le récipient est remplacé toutes les 2 heures, de sorte à renouveler périodiquement le milieu de culture contenu dans ledit récipient (1).

5. Procédé selon l'une quelconque des revendications 1 à 3, **caractérisé en ce que** le récipient de culture (1) comprend au moins un orifice d'entrée (2) et au moins un orifice de sortie (3), le remplacement du milieu de culture étant réalisé par établissement d'un flux continu de milieu de culture provenant de la source (5) de milieu, ledit flux alimentant le récipient de culture (1) par l'intermédiaire de l'orifice d'entrée (2) et étant retiré dudit récipient de culture par l'intermédiaire de l'orifice de sortie (3), de sorte à renouveler en continu le milieu de culture contenu dans ledit récipient.

6. Procédé selon la revendication 5, **caractérisé en ce qu'**il comprend une phase stationnaire dans laquelle le flux est agencé pour maintenir constant le volume de milieu de culture contenu dans le récipient de culture (1).

7. Procédé selon la revendication 6, **caractérisé en ce que**, préalablement à la phase stationnaire, le flux est agencé pour augmenter le volume initial de milieu de culture.

8. Procédé selon l'une quelconque des revendications 5 à 7, **caractérisé en ce que** la durée d'établissement du flux est supérieure à 24 heures, notamment comprise entre 24 heures et 72 heures.

9. Procédé selon l'une quelconque des revendications 5 à 8, **caractérisé en ce que** le débit du flux alimentant l'orifice d'entrée (2) est compris entre 0,2 et 5 mL/minutes.

10. Procédé selon l'une quelconque des revendications 5 à 9, **caractérisé en ce que** le flux de milieu de culture est oxygéné préalablement à son alimentation dans l'orifice d'entrée (2).

11. Procédé selon l'une quelconque des revendications 5 à 10, **caractérisé en ce qu'**au moins une partie du flux de milieu de culture alimentant l'orifice d'entrée (2) provient du flux de milieu de culture qui a été préalablement retiré par l'orifice de sortie (3).

12. Procédé selon l'une quelconque des revendications 1 à 11, **caractérisé en ce que** le récipient de culture (1) est formé d'une poche souple empêchant l'adhésion des îlots pancréatiques.

13. Procédé selon l'une quelconque des revendications 1 à 12, **caractérisé en ce que** le récipient de culture (1) comprend au moins un déflecteur (4) disposé sur le chemin d'écoulement direct entre l'orifice d'entrée (2) et l'orifice de sortie (3) du récipient de culture (1).

14. Procédé selon l'une quelconque des revendications 5 à 13, **caractérisé en ce que** l'orifice d'entrée (2) du récipient de culture (1) est relié par l'intermédiaire d'une première tubulure (6) à un récipient (5) formant source de milieu de culture, une première tête de pompe péristaltique (13) étant associée à ladite première tubulure (6) de sorte à alimenter l'orifice d'entrée (2) avec le flux de milieu de culture provenant du récipient source (5).

15. Procédé selon la revendication 14, **caractérisé en ce que** l'orifice de sortie (3) du récipient de culture (1) est relié par l'intermédiaire d'une deuxième tubulure (10) à un récipient (9) destiné à contenir le milieu de culture retiré par l'orifice de sortie (3), une deuxième tête de pompe péristaltique (14) étant associée à la deuxième tubulure (10) de sorte à retirer du récipient (1) le flux de milieu de culture par l'orifice de sortie (3).

16. Procédé selon la revendication 15, **caractérisé en ce qu'**une troisième tubulure (15) est branchée entre les première et deuxième tubulures (6,10), de sorte qu'au moins une partie du flux s'écoulant dans la deuxième tubulure (10) soit réintroduit dans la première tubulure (6).

17. Procédé selon les revendications 10 et 14, **caractérisé en ce qu'**un système d'aération (24) du flux de milieu de culture est placé sur le chemin d'écoulement de la première tubulure (1).

## Patentansprüche

1. Verfahren zum Konservieren pankreatischer Inselzellen, die dazu bestimmt sind, transplantiert zu werden, das die folgenden Schritte umfasst:
- das Einführen eines anfänglichen Volumens an Nährmedium und pankreatischer Inselzellen in ein Kulturgefäß (1),
- das Vorsehen einer Quelle (5) von Nährmedium,
- das Ersetzen in zeitlichen Abständen, die kleiner sind als 8 Stunden, des Nährmediums des Kulturgefässes durch Nährmedium, das aus der Quelle stammt, derart, dass das Nährmedium, das in dem Gefäß enthalten ist, erneuert wird, wobei das Verfahren das Erreichen einer Überlebensrate größer als 60 % bis zu 72 Stunden erlaubt.

2. Verfahren nach Anspruch 1, **dadurch gekennzeichnet, dass** die Konzentration der pankreatischen Inselzellen in dem Nährmedium zwischen 400 und 40.000 Inselzellen-Äquivalente/ml ist.

3. Verfahren nach einem der Ansprüche 1 oder 2, **dadurch gekennzeichnet, dass** das Nährmedium ein Basismedium umfasst, das mit Albumin angereichert ist.

4. Verfahren nach einem der Ansprüche 1 bis 3, **dadurch gekennzeichnet, dass** das Nährmedium, das in dem Gefäß enthalten ist, in Abständen von 2 Stunden derart ersetzt wird, dass das Nährmedium, das in dem Gefäß (1) enthalten ist, periodisch erneuert wird.

5. Verfahren nach einem der Ansprüche 1 bis 3, **dadurch gekennzeichnet, dass** das Kulturgefäß (1) mindestens eine Eingangsöffnung (2) und mindestens eine Ausgangsöffnung (3) umfasst, wobei das Ersetzen des Nährmediums durch Herstellen eines kontinuierlichen Flusses von Nährmedium, das von der Quelle (5) von Nährmedium stammt, ausgeführt wird, wobei der Fluss, der das Kulturgefäß (1) über die Eingangsöffnung (2) versorgt, aus dem Kulturgefäß über die Ausgangsöffnung (3) derart entnommen wird, dass das Kulturmedium, das in dem Gefäß enthalten ist, kontinuierlich erneuert wird.

6. Verfahren nach Anspruch 5, **dadurch gekennzeichnet, dass** es eine stationäre Phase umfasst, in der der Fluss eingerichtet ist, um das Volumen des Nährmediums, das in dem Kulturgefäß (1) enthalten ist, konstant zu halten.

7. Verfahren nach Anspruch 6, **dadurch gekennzeichnet, dass** der Fluss vor der stationären Phase eingerichtet ist, um das anfängliche Volumen an Nährmedium zu erhöhen.

8. Verfahren nach einem der Ansprüche 5 bis 7, **dadurch gekennzeichnet, dass** die Dauer des Erstellens des Flusses größer ist 24 Stunden, insbesondere zwischen 24 Stunden und 72 Stunden.

9. Verfahren nach einem der Ansprüche 5 bis 8, **dadurch gekennzeichnet, dass** der Durchsatz des Flusses, der die Eingangsöffnung (2) versorgt, zwischen 0,2 und 5 ml/Minute liegt.

10. Verfahren nach einem der Ansprüche 5 bis 9, **dadurch gekennzeichnet, dass** der Fluss an Nährmedium vor seiner Zuführung in die Eingangsöffnung (2) mit Sauerstoff angereichert wird.

11. Verfahren nach einem der Ansprüche 5 bis 10, **dadurch gekennzeichnet, dass** mindestens ein Teil des Flusses an Nährmedium, der die Eingangsöffnung (2) versorgt, von dem Fluss an Nährmedium stammt, der zuvor von der Ausgangsöffnung (3) entnommen wurde.

12. Verfahren nach einem der Ansprüche 1 bis 11, **dadurch gekennzeichnet, dass** das Kulturgefäß (1) aus einem geschmeidigen Beutel geformt ist, der das Haften der pankreatischen Inselzellen verhindert.

13. Verfahren nach einem der Ansprüche 1 bis 12, **dadurch gekennzeichnet, dass** das Kulturgefäß (1) mindestens einen Ablenker (4) umfasst, der auf dem direkten Fließweg zwischen der Eingangsöffnung (2) und der Ausgangsöffnung (3) des Kulturgefässes (1) liegt.

14. Verfahren nach einem der Ansprüche 5 bis 13, **dadurch gekennzeichnet, dass** die Eingangsöffnung (2) des Kulturgefässes (1) über einen ersten Rohrstutzen (6) mit einem Behälter (5) verbunden ist, der die Nährmediumquelle bildet, wobei ein erster Kopf einer peristaltischen Pumpe (13) mit dem ersten Rohrstutzen (6) derart verbunden ist, dass die Eingangsöffnung (2) mit dem Fluss an Nährmedium, der von dem Quellengefäß (5) stammt, versorgt wird.

15. Verfahren nach Anspruch 14, **dadurch gekennzeichnet, dass** die Ausgangsöffnung (3) des Kulturgefässes (1) über einen zweiten Rohrstutzen (10) mit einem Behälter (9) verbunden ist, der dazu bestimmt ist, das Nährmedium zu enthalten, das von der Ausgangsöffnung (3) entnommen wurde, wobei ein zweiter Kopf einer peristaltischen Pumpe (14) mit dem zweiten Rohrstutzen (10) derart verbunden ist, dass der Fluss an Nährmedium aus dem Gefäß (1) durch die Ausgangsöffnung (3) entnommen wird.

16. Verfahren nach Anspruch 15, **dadurch gekennzeichnet, dass** ein dritter Rohrstutzen (15) zwischen dem ersten und dem zweiten Rohrstutzen (6, 10) derart angeschlossen ist, dass mindestens ein Teil des Flusses, der aus dem zweiten Rohrstutzen (10) fließt, wieder in den ersten Rohrstutzen (6) eingeführt wird.

17. Verfahren nach den Ansprüchen 10 und 14, **dadurch gekennzeichnet, dass** ein Belüftungssystem (24) des Nährmediumflusses auf dem Fließweg des ersten Rohrstutzens (1) platziert ist.

## Claims

1. Process for preserving pancreatic islets intended to be transplanted, including the following steps:
- introducing an initial volume of culture medium and pancreatic islets into a culture container (1),
- providing a culture medium source (5),
- replacing, at time intervals below 8 hours, the culture medium in said culture container with culture medium from said source so as to renew the culture medium contained in said container, said process enabling a survival rate of above 60% to be obtained for up to 72 hours.

2. Process according to claim 1, **characterized in that** the concentration of the pancreatic islets in the culture medium is between 400 and 40 000 islet equivalents/mL.

3. Process according to claim 1 or 2, **characterized in that** the culture medium includes a base medium supplemented with albumin.

4. Process according to any one of claims 1 to 3, **characterized in that** the culture medium contained in the container is replaced every 2 hours so as to periodically renew the culture medium contained in said container (1).

5. Process according to any one of claims 1 to 3, **characterized in that** the culture container (1) includes at least one inlet orifice (2) and at least one outlet orifice (3), the replacement of the culture medium being performed by establishing a continuous flow of culture medium coming from the medium source (5), said flow supplying the culture container (1) by means of the inlet orifice (2) and being removed from said culture container by means of the outlet orifice (3), so as to continuously renew the culture medium contained in said container.

6. Process according to claim 5, **characterized in that** it includes a stationary phase in which the flow is arranged to keep the volume of culture medium contained in the culture container (1) constant.

7. Process according to claim 6, **characterized in that**, prior to the stationary phase, the flow is arranged to increase the initial volume of culture medium.

8. Process according to any one of claims 5 to 7, **characterized in that** the period for establishing the flow is greater than 24 hours, and in particular between 24 hours and 72 hours.

9. Process according to any one of claims 5 to 8, **characterized in that** the flow rate of the flow supplying the inlet orifice (2) is between 0.2 and 5 mL/minute.

10. Process according to any one of claims 5 to 9, **characterized in that** the flow of culture medium is oxygenated before being supplied to the inlet orifice (2).

11. Process according to any one of claims 5 to 10, **characterized in that** at least some of the flow of culture medium supplying the inlet orifice (2) comes from the flow of culture medium that has previously been removed through the outlet orifice (3).

12. Process according to any one of claims 1 to 11, **characterized in that** the culture container (1) is formed by a flexible pouch preventing adhesion of the pancreatic islets.

13. Process according to any one of claims 1 to 12, **characterized in that** the culture container (1) includes at least one deflector (4) arranged on the direct flow path between the inlet orifice (2) and the outlet orifice (3) of the culture container (1).

14. Process according to any one of claims 5 to 13, **characterized in that** the inlet orifice (2) of the culture container (1) is connected by means of a first tubing (6) to a container (5) forming a culture medium source, a first peristaltic pump head (13) being associated with said first tubing (6) so as to supply the inlet orifice (2) with the flow of culture medium coming from said source container (5).

15. Process according to claim 14, **characterized in that** the outlet orifice (3) of the culture container (1) is connected by means of a second tubing (10) to a container (9) intended to contain the culture medium removed through the outlet orifice (3), a second peristaltic pump head (14) being associated with the second tubing (10) so as to remove from the container (1) the flow of culture medium through the outlet orifice (3).

16. Process according to claim 15, **characterized in that** a third tubing (15) is connected between the first and second tubings (6, 10), so that at least some of the flow flowing into the second tubing (10) is reintroduced into the first tubing (6).

17. Process according to claims 10 and 14, **characterized in that** a system (24) for aeration of the flow of culture medium is placed on the flow path of the first tubing (1).
